# EUROPEAN PATENT APPLICATION

(11) **EP 3 437 722 A1**
(43) Date of publication of application: **06.02.2019**
(21) Application number: 17775504.8
(22) Date of filing: 30.03.2017
(51) Int. Cl.: B01D 63/02, A61M 1/18, B01D 63/00

(54) **HOLLOW FIBER MEMBRANE MODULE AND METHOD FOR MANUFACTURING SAME**

(30) Priority: 31.03.2016 JP 2016073226
(71) Applicant: Asahi Kasei Medical Co., Ltd., Chiyoda-ku Tokyo 101-8101 (JP)
(72) Inventor: ASATSUMA, Keiichi, Tokyo 10-18101 (JP); KOIZUMI, Toshinori, Tokyo 101-8101 (JP); KOYANO, Toshihiro, Tokyo 101-8101 (JP)
(74) Representative: dompatent von Kreisler Selting Werner - Partnerschaft von Patent- und Rechtsanwälten mbB
(86) International application number: PCT/JP2017/013490
(87) International publication number: WO 2017/170971

(57) **Abstract**

The hollow fiber membrane module according to the present application is provided with a cylindrical container (2) open at one end and the other end, a bundle of hollow fiber membranes (3) packed in the cylindrical container (2), potting parts (4) embedding and fixing the bundle of hollow fiber membranes (3) at both ends of the cylindrical container (2), and headers (5) provided at both ends of the cylindrical container (2) and having nozzles that constitute an inlet and outlet for a fluid. In this hollow fiber membrane module, the headers (5) and the cylindrical container (2) are welded in at least two areas including a primary weld (71) and a secondary weld (72), and the secondary weld (72) is located in a position where welding is initiated after or simultaneously with the beginning of welding of the primary weld (71).

## Description

### Technical Field

The present invention relates to a hollow fiber membrane module that purifies blood using a hollow fiber membrane packed in a container body, and to a manufacturing method therefor.

### Background Art

Conventionally, various hollow fiber membrane-type blood purifiers (called "hollow fiber membrane modules" in this Description) have been developed for extracorporeal blood purification therapies such as hemodialysis, blood filtration, plasma separation and plasma component fractionation, and these are applied in many blood purification therapies and the like using membrane separation technology.

A hollow fiber membrane module is generally constituted by a module formed by packing a bundle of hollow fiber membranes in a cylindrical container body having a port on the side, adhesively fixing the hollow fiber membrane bundle to the container body by a potting material such as urethane, and attaching headers to both ends of the container body. To perform hemodialysis using this hollow fiber membrane module, a dialysate is passed through the container body by causing the dialysate to flow into a port on the inlet side and discharging the dialysate through an outlet port, while blood is introduced into the hollow fiber membranes from the header on the blood inlet side, and made to flow toward the header on the blood outlet side.

When performing hemodialysis in this way, a liquid-tight seal is necessary to prevent liquid from leaking out from the junction between the header and the container body, and a method of joining the header and container body by ultrasonic welding is used for example. In this method, the part of the header to be welded and the part of the container to be welded are made to abut one another as ultrasonic vibration is applied to bond the two by heating and melting, and this is a preferred method for obtaining liquid-tight and air-tight seals (see for example Patent Document 1).

### Citation List

### Patent Document

Patent Document 1: WO 2013/146663

### Summary

### Technical Problem

However, the bonding strength between the header and container body of the blood purification unit is not always satisfactory, and if the bonding strength is insufficient the joint may be disengaged by shock during transport. Moreover, insufficient bonding strength leads to insufficient pressure-resistant strength, which can cause the joint to disengage and leak liquid during blood purification therapy.

It is an object of the present invention, which was developed to solve these problems, to provide a hollow fiber membrane module whereby the problems of insufficient bonding strength, insufficient pressure-resistant strength and liquid leakage have been solved by improving the bonding strength between the header and the container body.

### Solution to Problem

To solve these problems, the present invention is a hollow fiber membrane module including:
a cylindrical container open at one end and the other end;
a bundle of hollow fiber membranes packed in the cylindrical container;
potting parts embedding and fixing the bundle of hollow fiber membranes at both ends of the cylindrical container; and
headers provided at both ends of the cylindrical container and having nozzles that constitute an inlet and outlet for a fluid, wherein
the headers and the cylindrical container are welded in at least two areas including a primary weld and a secondary weld, and
the secondary weld is located in a position where welding is initiated after or simultaneously with the beginning of welding of the primary weld.

While headers and cylindrical containers have been welded in only one area in the past, in the hollow fiber membrane module of this Description the bonding strength of the hollow fiber membrane module is increased and pressure-resistant strength is improved because of the welding in at least two areas. Consequently, the pressure-resistant strength is unlikely to be insufficient, and liquid leakage is unlikely.

In the hollow fiber membrane module, the melted length of a melted part of the primary weld, which is a length parallel to the central axis of the cylindrical container, is preferably either longer than or the same length as the weld length of a melted part of the secondary weld.

A recess into which a part of each of the headers fits may be formed in the cylindrical container.

This recess may be formed by a flange that projects outward from an outer surface of the cylindrical container and is bent toward each of the headers.

The primary weld is preferably located on the side nearer to a junction surface between each of the headers and a welding horn than the secondary weld and other welds.

In the hollow fiber membrane module, either or both of the primary weld and the secondary weld may use a share joint as a joint design for welding each of the headers to the cylindrical container.

The present invention is also a method for manufacturing a hollow fiber membrane module including:
a cylindrical container open at one end and the other end;
a bundle of hollow fiber membranes packed in the cylindrical container;
potting parts embedding and fixing the bundle of hollow fiber membranes at both ends of the cylindrical container; and
headers provided at both ends of the cylindrical container and having nozzles that constitute an inlet and outlet for a fluid,
the method including:
welding the headers and the cylindrical container in at least two areas including a primary weld and a secondary weld; and
initiating welding of the secondary weld after or simultaneously with the beginning of welding of the primary weld.

In the method for manufacturing a hollow fiber membrane module, the weld length of the primary weld parallel to the central axis of the cylindrical container is preferably longer than or the same as the weld length of the secondary weld.

### Advantageous Effects of Invention

With the present invention, it is possible to improve the bonding strength between the headers and container body of a hollow fiber membrane module, and resolve problems of insufficient bonding strength, insufficient pressure-resistant strength and liquid leakage.

### Brief Description of Drawings

Fig. 1 is a vertical cross-section showing one example of the configuration of a hollow fiber membrane module.
Fig. 2 is an enlarged cross-section view showing the junctions and the areas around the junctions between a header and cylindrical container at the beginning of welding by ultrasonic horn.
Fig. 3 is a cross-section showing ultrasonic welding of a header and cylindrical container.
Fig. 4 is an enlarged cross-section showing the junctions and the areas around the junctions of the welded header and cylindrical container.

### Description of Embodiments

Preferred embodiments of the present invention are explained below with reference to the drawings. The same elements are shown with the same symbols, and duplicate explanations are omitted. Unless otherwise specified, positional relationships such as up and down and left and right are based on the positional relationships in the drawings. The dimensional ratios in the drawings are not limited to the ratios shown. Moreover, the embodiments below are examples used to illustrate the invention, and the invention is in no way limited by these embodiments.

### <Configuration of hollow fiber membrane module>

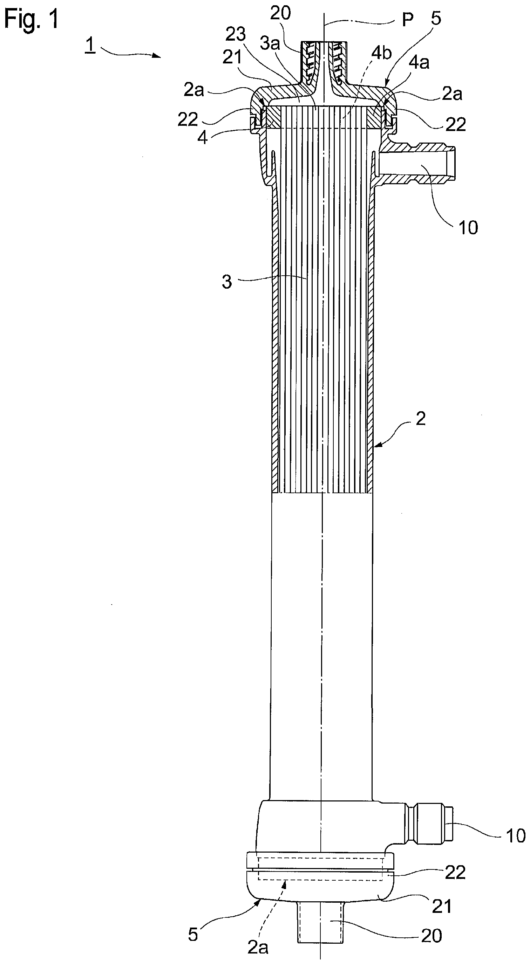

The configuration of the hollow fiber membrane module in these embodiments is explained first. Fig. 1 is a cross-sectional view (showing only the cross-section) of one example of the configuration of a hollow fiber membrane module 1.

The hollow fiber membrane module 1 includes cylindrical container 2, hollow fiber membrane bundle 3, potting part 4 and headers 5.

The cylindrical container 2 is formed as a cylinder, and is open at both ends 2a in the longitudinal direction (direction of central axis P of cylinder). The hollow fiber membrane bundle 3 is contained within the cylindrical container 2. Two ports 10 for example are formed in the side of the cylindrical container 2 as an outlet and inlet for fluid.

The hollow fiber membrane bundle 3 is a bundle of multiple hollow fiber membranes, which are aligned in the longitudinal direction of the cylindrical container 2. The hollow fiber membrane bundle 3 functions as a separation membrane, and components of a fluid to be separated are separated between an inner region and outer region of each hollow fiber membrane.

The potting part 4 is made of a potting resin, and embeds both ends 3a of the hollow fiber membrane bundle 3 within both ends 2a of the cylindrical container 2 while fixing the hollow fiber membrane bundle 3 to both ends 2a of the cylindrical container 2. The outer periphery of the potting part 4 constitutes a part 4a made only of potting resin, and the inner side of the potting part 4 constitutes a part 4b in which the potting resin fills the gaps between the hollow fiber membranes of the hollow fiber membrane bundle 3. Examples of the potting resin include, but are not limited to, polyurethane resin, epoxy resin, silicone resin and the like.

The headers 5 are provided at the openings of the ends 2a of the cylindrical container 2 as caps for these ends 2a. The headers 5 have tubular nozzle parts 20 on central axis P to serve as an inlet and outlet for fluid, plate-shaped top plates 21 extending radially from the nozzle parts 20, and header projections 22 projecting as side walls from the outer periphery of the top plates 21 toward the cylindrical container 2.

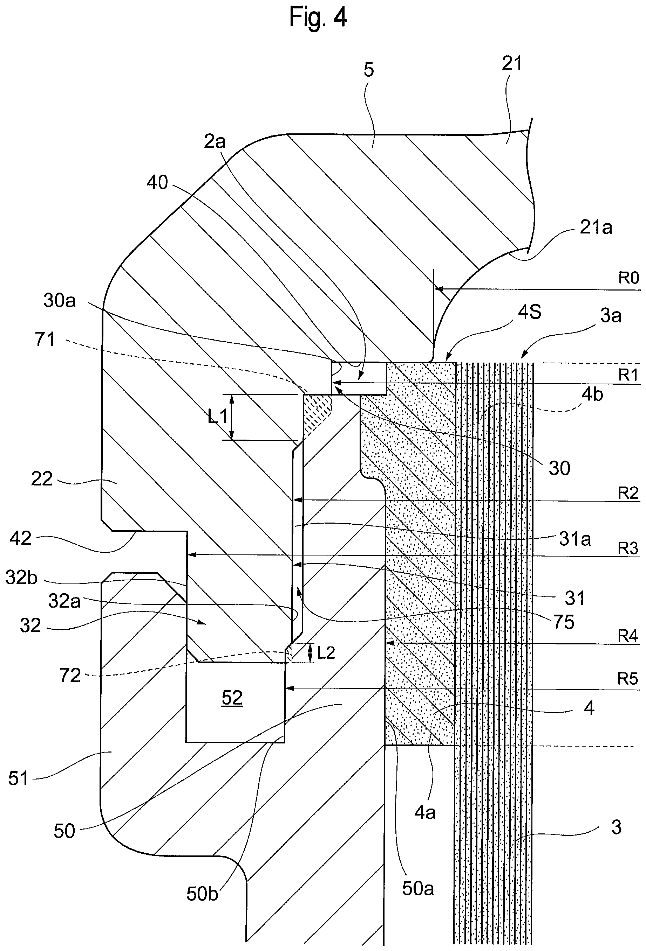

Each nozzle part 20 has a screw structure for connecting to an external tube. The top plates 21 have inner surfaces 21a that face the ends 3a of the hollow fiber membrane bundle 3 and increase gradually in diameter from the nozzle parts 20 to the ends 2a of the cylindrical container 2 (see Fig. 4). The gap between the inner surface 21a of each header 5 and the end 3a of the hollow fiber membrane bundle 3 forms a space 23 through which flows a fluid flowing in from a nozzle part 20 or a fluid flowing out through a nozzle part 20.

The header projections 22 have a cylindrical shape with the central axis P as the central axis of the cylinder. As shown in Fig. 4 for example, each header projection 22 is provided with a base 30, a middle stage 31 and a tip 32 extending in that order from the top plate 21 toward the cylindrical container 2. The base 30, middle stage 31 and tip 32 each have a different thickness in the radial direction, with the base 30 being the thickest, the middle stage 31 the next thickest and the tip 32 the thinnest.

The base 30 is the part that is continuous with the top plate 21, and has for example a first inner peripheral surface 30a having a fixed inner diameter R1. The inner diameter R1 of the first inner peripheral surface 30a is greater than the inner diameter R0 of the bottom end (maximum diameter part) of the inner surface 21a of the top plate 21. An annular flat surface (step) 40 is formed perpendicular to the central axis P in the space between the inner surface 21a of the top plate 21 and the first inner peripheral surface 30a of the base 30.

The middle stage 31 has a second inner peripheral surface 31a having a fixed inner diameter R2 that is larger than the inner diameter R1. The tip 32 has a third inner peripheral surface 32a having the same inner diameter R2 as the second inner peripheral surface 31a and an outer peripheral surface 32b having a fixed outer diameter R3 that is smaller than the outer diameter of the outer peripheral surface 31b of the middle stage 31. An annular flat surface (stage) 42 is formed perpendicular to the central axis P in the space between the outer peripheral surface 31b of the middle stage 31 and the outer peripheral surface 32b of the tip 32.

The ends 2a of the cylindrical container 2 have a so-called bifurcated structure. Each end 2a has an inner projection 50 and an outer projection 51 that project in the form of a double cylinder toward the header 5. The inner projection 50 and outer projection 51 each have a cylindrical shape with the central axis P as the central axis of the cylinder, and are arranged concentrically. The inner projection 50 projects further than the outer projection 51 toward the header 5 side (outer side in the direction of central axis P). The inner peripheral surface 50a of the inner projection 50 constitutes the inner surface of the cylindrical container 2.

The outer projection 51 is formed by a flange that projects radially outward from the outer surface of the cylindrical container 2 and is then bent from the outer edge toward the end 2a (toward the nearest header 5). An annular recess 52 constituting a coupling structure is formed so that a part of the header 5 (tip 32) fits between the inner projection 50 and the outer projection 51 (see Fig. 4).

The inner peripheral surface 50a of the inner projection 50 has an inner diameter R4 that is larger than the inner diameter R0 and smaller than the inner diameter R1. The outer peripheral surface 50b of the inner projection 50 has an outer diameter R5 that is larger than the inner diameter R1 and smaller than the inner diameter R2.

### <Junction structure of header and cylindrical container

The header 5 and cylindrical container 2 are welded by ultrasonic welding with the header projection 22 inserted between the inner projection 50 and outer projection 51 of the cylindrical container 2. The inner projection 50 faces the middle stage 31 and tip 32 of the header projection 22, while the outer projection 51 faces the tip 32.

The header 5 and cylindrical container 2 are welded by weld parts 70 formed of a primary weld 71 between the middle stage 31 of the header projection 22 and an area near the end of the inner projection 50 and a secondary weld 72 between the inner projection 50 and the tip 32. It is thus possible to improve the leakage prevention effect and pressure-resistant strength by means of a double weld formed of welds located in at least two areas.

The primary weld 71 is formed for example by the end face (upper end face in Fig. 4) and the outer peripheral surface 50b of the inner projection 50. In Fig. 4, the primary weld 71 is represented by broken lines and has a definite outline, but in actually welding where the contacting parts melt and adhere, the boundaries are indistinct and differ from weld to weld.

The secondary weld 72 is formed for example near the end of the tip 32 of the header projection 22 (closer to the end than the center of the tip 32 in the longitudinal direction) (see Fig. 4). The secondary weld 72 is separated from the primary weld 71, and a space (indicated by the reference numeral 75 in Fig. 4) in which header projection 22 and inner projection 50 do not contact one another is formed between the secondary weld 72 and the primary weld 71.

In order for welding to occur in the locations of the primary weld 71 and the secondary weld 72, either one or both of the header projection 22 and the inner projection 50 are provided with a welding allowance (interference part) in the parts that will become the primary weld 71 and secondary weld 72.

Moreover, at least one of the primary weld 71 and secondary weld 72 is formed continuously around the entire circumference of the header projection 22, while the other is formed discontinuously for example. More preferably, both the primary weld 71 and the secondary weld 72 are formed continuously around the entire circumference.

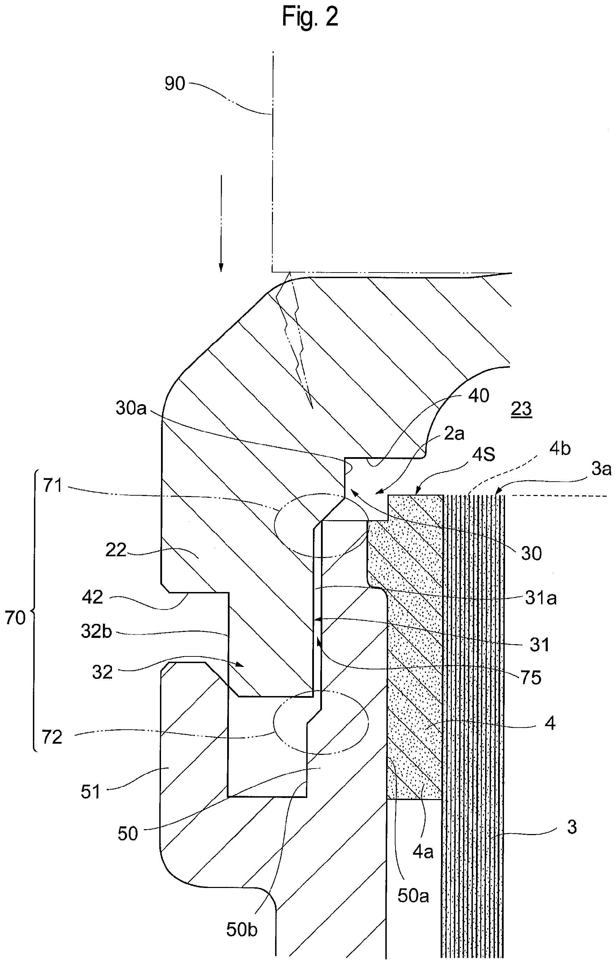

Furthermore, in the hollow fiber membrane module 1 of this embodiment, the secondary weld 72 is located in a position where welding is initiated either after or simultaneously with the start of welding of the primary weld 71 (see Fig. 2). In general, depending on the direction of insertion of the header 5 into (recess 52 of) the cylindrical container 2 during ultrasonic welding, it may not be possible to ensure an adequate melt volume for both primary and secondary welding, and adequate bonding strength and pressure-resistant strength may not be obtained. In particular, in a weld such as the secondary weld 72 of this embodiment in which the tip 32 of the header 5 is inserted into the recess 52 (coupling structure), the direction of entry of the coupling structure tends to shift, affecting the welding of the primary weld 71 and making it impossible to ensure an adequate weld volume. Moreover, if (the part that will become) the secondary weld 72 is brought into contact first, (the part that will become) the primary weld 71 is welded after the tip 32 of the header 5 has opened outwardly, and the welding volumes of both the primary weld 71 and the secondary weld 72 may be reduced as a result (the reason why the tip 32 opens outwardly if the secondary weld 72 comes into contact first but not if the primary weld 71 comes into contact first is thought to be that the secondary weld 72 is further from the ultrasonic horn 90 and the vibration is not adequately transmitted). By contrast, if the welding of the secondary weld 72 is initiated either after or simultaneously with the start of welding of the primary weld 71 as in the present embodiment, it is easy to ensure an adequate welding volume of both the primary weld 71 and the secondary weld 72. In this case, the welding allowance (interference part) on the side of the tip 32 and the welding allowance (interference part) of the inner projection 50 that will constitute the secondary weld 72 come into contact as ultrasonic waves are emitted from the ultrasonic horn 90, and are welded under pressure. Since vibrations are better transmitted the closer the distance from the ultrasonic horn 90, stable welding can be achieved by welding in sequence from the position closest to the ultrasonic horn 90 (primary weld 71).

Moreover, in the hollow fiber membrane module 1 of this embodiment, the weld length L1 of the melted part of the primary weld 71 parallel to the central axis P is equal to or greater than the weld length L2 of the melted part of the secondary weld 72 (see Fig. 4).

Furthermore, the primary weld 71 is located closer than the other welded parts (secondary weld 72 in this embodiment) to the junction surface between the header 5 and the ultrasonic horn (welding horn) 90 (see Fig. 2). The junction surface between the header 5 and the ultrasonic horn 90 is the surface of the top plate 21 of the header 5 which contacts the ultrasonic horn 90 during ultrasonic bonding (see Fig. 2). The primary weld 71 and the other welded parts (secondary weld 72 in this embodiment) may be each be located at different distances from the central axis P. Moreover, the primary weld 71 may be located closer to the central axis P that the other welded parts (secondary weld 72 in this embodiment).

In this embodiment, a share joint is adopted as the joint design of the primary weld 71 and secondary weld 72 for welding the header 5 and the cylindrical container 2 (see Fig. 4), but this is only a preferred example. One advantage of a share joint is that because the contact surfaces and vibrational directions of each member (the header 5 and cylindrical container 2) are in nearly the same direction relative to the longitudinal vibration of the ultrasonic horn 90, bubbles are less likely to form on the welded surface, resulting in excellent liquid-tightness. Another advantage is that because the vibration acts like rubbing on the edge parts and taper parts (see parts in ovals in Fig. 2; the edge-like angular parts and tapered parts in primary weld 71 and secondary weld 72), the heating effect is good and high welding strength can be obtained. Although this is not shown, a butt joint can also be adopted as the joint design for the primary weld 71 and secondary weld 72. When ultrasonic welding a crystalline resin, a highly liquid-tight weld can be achieved even using a butt joint if the work size is small. As the work size increases, however, weldability may decrease due to attenuation of the ultrasonic waves as the distance between the ultrasonic horn 90 and the welded surface increases. Consequently, if a butt joint is adopted for either the primary weld 71 or the secondary weld 72, it is preferably adopted for the primary weld 71 (which is closer to the ultrasonic horn 90) rather than for the secondary weld 72.

### <Arrangement of Potting Part>

As shown in Fig. 4, potting part section faces 4S are formed as the end faces of the potting part 4 by cutting both ends of the potting part in the radial direction. The potting part 4 projects further toward the outside (header 5 side) in the central axis P direction than does the inner projection 50 of the cylindrical container 2. The potting part section face 4S abuts the flat surface 40 on the inner surface of the header 5 under a specific degree of pressure. It is thus possible to prevent liquid from easily infiltrating the gap between the header projection 22 and the end 2a of the cylindrical container 2, and to improve the sealing power between the header 5 and the cylindrical container 2.

The raw materials of the cylindrical container 2 and header 5 are not particularly limited, and are selected from various thermoplastic resins. Examples of crystalline resins include copolymers of ethylene and α-olefins, polyethylene resins such as low-density polyethylene and high-density polyethylene, and polypropylene resins including polymers of propylene monomers, copolymers of propylene with ethylene, and copolymers of propylene and ethylene with other α-olefins. In terms of amorphous resins, examples include polyester, polycarbonate, polystyrene, styrenebutadiene copolymer (SBS) and acrylonitrile-butadiene-styrene copolymer (ABS), and one of these alone or a mixture thereof may be used. In the present embodiment, a polypropylene resin is preferred out of those listed above, a random copolymer of propylene and ethylene is preferred from the standpoint of rigidity and heat resistance, and a random propylene-ethylene copolymer with an adjusted ethylene content of 1 to 8 mass% is especially desirable.

### <Method for manufacturing hollow fiber membrane module>

A hollow fiber membrane bundle 3 is first prepared with a length longer than the final required length, and enclosed in a cylindrical container 2. A potting part 4 is then formed inside the cylindrical container 2. The potting part 4 fixes the hollow fiber membrane bundle 3 to the inner peripheral surface 50a of the cylindrical container 2 while embedding both ends 3a of the hollow fiber membrane bundle 3. Next, the unneeded portions of the potting part 4 (ends 3a of hollow fiber bundle 3) are cut cross-sectionally perpendicular to the central axis P to form potting part section faces 4S.

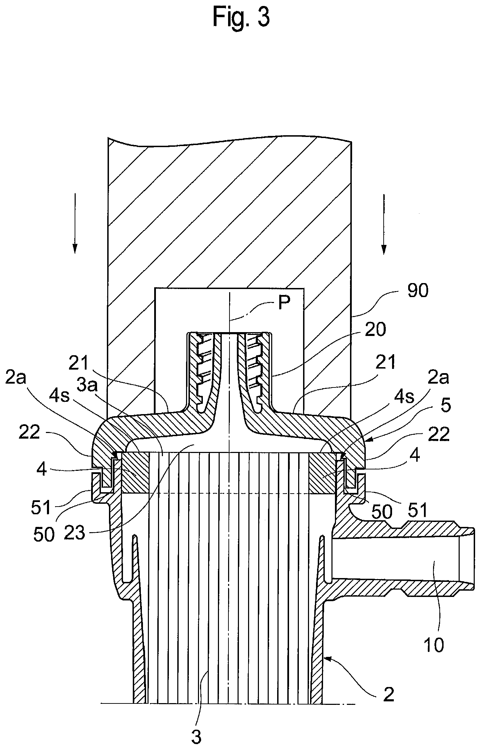

Next, the header 5 is welded to the cylindrical container 2. As shown in Fig. 3 for example, the header projection 22 of the header 5 is inserted between the inner projection 50 and outer projection 51 of the cylindrical container 2, while the potting part section face 4S is made to abut the flat surface 40 on the inner surface of the header 5. In this state, the ultrasonic horn 90 contacts the top plate 21 of the header 5 from the outside. When the ultrasonic horn 90 then presses the header 5 from the outside toward the cylindrical container 2 in the direction of the central axis P while emitting ultrasonic vibration toward the header 5, the primary weld 71 and secondary weld 72 are welded by ultrasonic welding (a technique whereby electrical energy is converted to mechanical vibration energy, and pressure is applied at the same time to generate strong heat at the junction surface between two parts to be welded, melting the plastic and bonding the two). In the hollow fiber membrane module 1 of this embodiment, welding of the secondary weld 72 is initiated after the start of welding of the primary weld 71 (see Fig. 2). Such sequential welding is given as an example in this embodiment, but it is also possible to adopt a welding method in which the primary weld 71 and secondary weld 72 are brought into contact simultaneously to induce a trigger. Depending on the ultrasonic horn 90, moreover, welding can be initiated after ultrasonic welding has already been generated.

The header 5 and cylindrical container 2 are thus welded in at least two places to complete the hollow fiber membrane module 1 (see Fig. 1). In the present embodiment, bonding strength is improved because the header 5 and cylindrical container 2 are welded in two places by the primary weld 71 and secondary weld 72, thereby improving the bonding strength and pressure-resistant strength of the hollow fiber membrane module 1. Consequently, pressure-resistant strength is unlikely to be inadequate, and liquid leakage is unlikely.

When the hollow fiber membrane module 1 is in use for example, a fluid such as blood is made to flow into a header 5 through a nozzle part 20, passes through each hollow fiber membrane of the hollow fiber membrane bundle 3, and is discharged from the nozzle part 20 of the opposite header 5, and during this process a specific component of the fluid is removed to the outside via the side walls of the hollow fiber membranes. Clogging of the hollow fiber membranes progresses with continued use of the hollow fiber membrane module 1, raising the internal pressure of the hollow fiber membrane module 1. Once the pressure of the fluid inside the header 5 rises beyond a certain point, there is a possibility that the fluid may escape to the outside between the potting part 4 and the inner surface of the header 5. In this case, fluid pressure acts on the primary weld 71 between the header projection 22 and the inner projection 50. Shearing force is thus applied to the header projection 22 toward of the cylindrical container 2 (downwards in Fig. 4), pushing the header 5 against the cylindrical container 2, with the result that the header 5 is prevented from separating from the cylindrical container 2. Even if the fluid pressure inside the header 5 rises still further and a part of the primary weld 71 is broken, the pressure then acts on the secondary weld 72 between the header projection 22 and the inner projection 50. The shearing force acting toward the cylindrical container 2 is thus continuously applied to the header projection 22, thereby preventing the header 5 from separating from the cylindrical container 2. As a result, the header 5 is unlikely to separate from the cylindrical container 2, and the pressure-resistant strength of the hollow fiber membrane module 1 is improved.

Looking at the pressure-resistant strength of the hollow fiber membrane module 1, because the strength of the welded part is greater in the primary weld 71 than in the secondary weld 72, it seems unlikely that the primary weld 71 would break before the secondary weld 72, but in fact if the pressure rises as described above, it could happen that the primary weld 71 would break before the secondary weld 72. Strengthening the primary weld 71 appears even more desirable since the results of a simulation have shown that stronger pressure acts on the primary weld 71 than on the secondary weld 72.

In fact, strength is increased more by welding both welded parts, the primary weld 71 and secondary weld 72, rather than by increasing the strength of only one welded part, and one reason for this is that pressure-resistant strength is increased by supporting the device at two locations (two support points), the primary weld 71 and the secondary weld 72. The strength in this case correlates with the weld lengths L1 and L2 of the primary weld 71 and secondary weld 72. Two-stage welding is required in order to increasing the weld lengths L1 and L2. Furthermore, increasing the weld length L1 is preferred because it increases the overall pressure-resistant strength. It is thought that the weld length L1 has a greater effect because it is located in a position that is vulnerable to the effects of pressure.

The embodiment described above is one example of a preferred embodiment of the present invention, but this does not limit the invention and various changes can be implemented to the extent that these do not deviate from the intent of the invention. For example, in the embodiment described above the header 5 and cylindrical container 2 are welded at two locations, the primary weld 71 and secondary weld 72, but of course it is also desirable to weld the header 5 and cylindrical container 2 at more locations, or in other words three or more locations. To give an example of welds other than the primary weld 71 and secondary weld 72 without giving specific details, the device could be welded in an area between the tip 32 and the outer projection 51 to form a tertiary weld (not shown).

Moreover, the structure of the hollow fiber membrane module 1 shown in the embodiment described above is only an example, and for example the structure of the header projection 22 of the header 5 and the structures of the inner projection 50 and outer projection 51 of the cylindrical container 2 are not limited to those described above. Moreover, the overall structures of the cylindrical container 2 and header 5 are not limited to those described above. Furthermore, the use of the hollow fiber membrane module 1 is not limited to treatment of liquids such as blood, and it may also be used to treat gases.

### Industrial Applicability

The present invention is useful for improving the pressure-resistant strength of a hollow fiber membrane module.

### Reference Signs List

- 1: Hollow fiber membrane module

- 2: Cylindrical container
- 2a: End
- 3: Hollow fiber membrane bundle
- 4: Potting part
- 4S: Potting part section face
- 5: Header
- 22: Header projection
- 50: Inner projection
- 51: Outer projection
- 52: Recess
- 70: Weld part
- 71: Primary weld
- 72: Secondary weld
- 90: Ultrasonic horn (welding horn)

## Claims

1. A hollow fiber membrane module comprising:
a cylindrical container open at one end and the other end;
a bundle of hollow fiber membranes packed in the cylindrical container;
potting parts embedding and fixing the bundle of hollow fiber membranes at both ends of the cylindrical container; and
headers provided at both ends of the cylindrical container and having nozzles that constitute an inlet and outlet for a fluid, wherein
the headers and the cylindrical container are welded in at least two areas including a primary weld and a secondary weld, and
the secondary weld is located in a position where welding is initiated after or simultaneously with the beginning of welding of the primary weld.

2. The hollow fiber membrane module according to claim 1, wherein the melted length of a melted part of the primary weld, which is a length parallel to the central axis of the cylindrical container, is either longer than or the same length as the weld length of a melted part of the secondary weld.

3. The hollow fiber membrane module according to claim 1 or 2, wherein a recess into which a part of each of the headers fits is formed in the cylindrical container.

4. The hollow fiber membrane module according to claim 3, wherein the recess is formed by a flange that projects outward from an outer surface of the cylindrical container and is bent toward each of the headers.

5. The hollow fiber membrane module according to any one of claims 1 to 4, wherein the primary weld is located on the side nearer to a junction surface between each of the headers and a welding horn than the secondary weld and other welds.

6. The hollow fiber membrane module according to any one of claims 1 to 5, wherein either or both of the primary weld and the secondary weld use a share joint as a joint design for welding each of the headers to the cylindrical container.

7. A method for manufacturing a hollow fiber membrane module including:
a cylindrical container open at one end and the other end;
a bundle of hollow fiber membranes packed in the cylindrical container;
potting parts embedding and fixing the bundle of hollow fiber membranes at both ends of the cylindrical container; and
headers provided at both ends of the cylindrical container and having nozzles that constitute an inlet and outlet for a fluid,
the method comprising:
welding the headers and the cylindrical container in at least two areas including a primary weld and a secondary weld; and
initiating welding of the secondary weld after or simultaneously with the beginning of welding of the primary weld.

8. The method for manufacturing a hollow fiber membrane module according to claim 7, wherein the weld length of the primary weld parallel to the central axis of the cylindrical container is longer than or the same as the weld length of the secondary weld.
